# EUROPEAN PATENT SPECIFICATION

(11) **EP 1 894 592 B1**
(45) Date of publication and mention of the grant of the patent: **24.03.2010**
(21) Application number: 07011256.0
(22) Date of filing: 08.06.2007
(51) Int. Cl.: A61M 16/16

(54) **An apparatus for adding humidity to gas**
Vorrichtung zum Hinzufügen von Feuchtigkeit zu Gas
Appareil pour ajouter de l'humidité à un gaz

(30) Priority: 31.08.2006 JP 2006265924
(43) Date of publication of application: 05.03.2008
(73) Proprietor: Sataco Co., LTD., Tokyo (JP)
(72) Inventor: Aonuma, Saburo, Shinagawa-ku Tokyo (JP)
(74) Representative: Wagner, Karl H.

(56) References cited:
- US-A- 4 381 267
- US-A- 5 738 808
- US-B1- 6 367 472

## Description

### BACKGROUND OF THE INVENTION

This invention relates to an apparatus for adding humidity to a subject gas for adding humidity.

There is an apparatus known for adding humidity to a subject gas such as air or nitrogen gas using a hollow-fiber membrane made from an ion-exchange membrane such as perfluoro system.

Japanese Patent Publication JP 08-276019 A describes a gas supplying device for breathing equipped with a humidifier without generating noise by bubble exploding sound, not like a bubble type humidifier, and with no leak of oxygen gas caused by imperfect handling.

In this patent publication, there are descriptions such as "humidity can be added to an oxygen gas to a desired degree of related humidity (such as 95% ∼100%)", and "the degree of humidity can be adjusted to a degree between a related humidity between 60% through 100% (preferably approximately 95% ∼ 100%)".

However, actually, in the structure described in this publication for adjusting humidity, only the volume of water 24 to be supplied from the water supply tank 22 should be controlled because the hollow-fiber membrane 4 is installed in an airtight container 5. In this publication there is description specifying "because the container 5 is in an airtight condition, the same volume of water consumed for adding humidity is supplied from the water supply tank 22." As a result, a constant volume of water is simply supplied to the container 5. Therefore, it is difficult to perform a fine-tuning at a degree of humidity to the subject gas for adding humidity.
US-A-5,738,808 was used as a basis for the preamble of claim 1 and discloses a humidifier for humidifying an inhalant gas, which does not include adjustment means for adjusting a pressure and/or a flow rate.

The present invention is provided in view of the above disadvantage. It is the object of the present invention to provide an apparatus for adding humidity to gas having function capable of adjusting degree of humidity to the subject gas easily and precisely.

The object of the present invention is achieved by an apparatus for adding humidity to gas as set forth in claim 1 and by a method as set forth in claims 6 or 7. Preferred embodiments of the present invention may be gathered from the dependent claims.

According to the apparatus for adding humidity to gas of the present invention, the water element containing liquid can be maintained at a specified surface level in contact with the hollow-fiber membrane and the internal ambient in the closed enclosure. The pressures and flow rates adjustment means are provided for adjusting pressures and flow rates of the subject gas to be supplied into the hollow membrane and the closed enclosure separately. As a result, the degree of humidity to the subject gas for adding humidity can be adjusted easily and precisely.

### BRIEF DESCRIPTION OF THE DRAWINGS

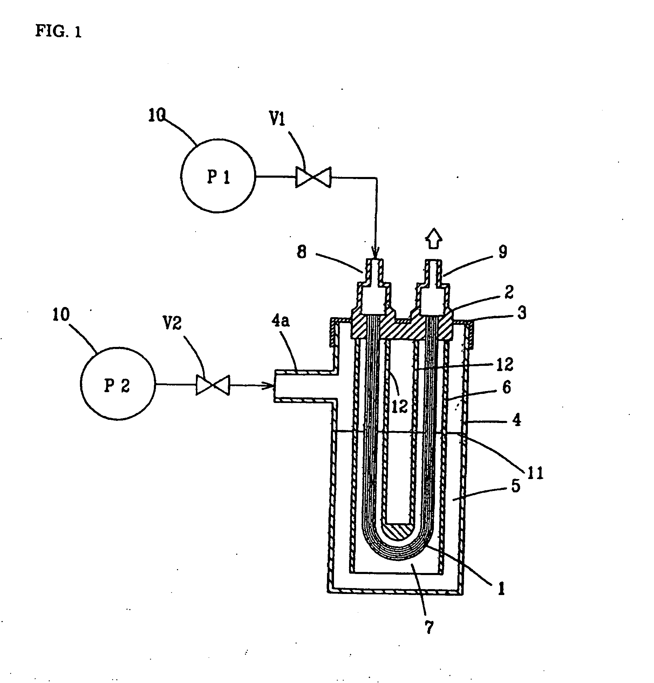

FIG. 1 is a sectional view showing an apparatus for adding humidity to gas according to the present invention.

### A PREFERRED EMBODIMENT OF THE INVENTION

The above and other objects, features and advantages of this invention and the manner of attaining them will become more apparent, and the invention itself will best be understood, from a study of the following description of a preferred embodiments illustrated in the attached drawings.

An embodiment of the present invention is described in details hereunder by referring to the drawing. FIG. 1 is a sectional view showing an apparatus for adding humidity to gas according to the present invention. According to the embodiment of the present invention, an apparatus for adding humidity to a subject gas includes a hollow-fiber membrane 1 and a closed enclosure 4 and the pressures and flow rates adjustment means are provided for adjusting pressures and flow rates of internal pressure in the closed enclosure 4, and adjusting pressures and flow rates of the subject gas into the hollow-fiber membrane 1. In particular, according to the embodiment of the present invention, the pressures and flow rates adjustment means are composed of pumps 10 (P1, P2) having an adjustable function for adjusting pressures, and valves (V1, V2) connected to each pumps for adjusting flow rates.

Fig. 1 is a sectional view showing an example of an apparatus for adding humidity to a subject gas according to the present invention. In Fig. 1, the apparatus for adding humidity to the subject gas having the hollow-fiber membrane 1 made from perfluoro system or an ion-exchange membrane etc. bent in U-character shape, a receptacle 2 made from an epoxy resin connected to the both ends of the hollow-fiber membrane 1, a cap member 3 made from an epoxy resin attached the receptacle thereon, a closed enclosure 4 made from an epoxy resin maintaining a water element containing liquid therein at a desired level, an electrolyzed water 5 as a water element containing liquid maintained in contact with the hollow-fiber membrane, an external sleeve 6 covering the hollow-fiber membrane 1 having openings 12 at upper portion thereof, the inlet 8 of the receptacle 2 for supplying a compressed gas to the hollow-fiber membrane from the pump 10 (P1) via the valve (V1), and the closed enclosure 4 having the inlet 4a for supplying a compressed gas from the pump 10 (P2) via the valve (V2).

The pumps 10 (P1, P2) can be provided separately or the pumps can be a single unit, however, a plurality of valves (V1, V2) may be provided to each path connecting with the single pump respectively.

Thus, pressures from the pumps 10 (P1, P2) are adjusted to specified pressures by adjusting their outputs, and flow rates are adjusted to specified flow rates using the valves (V1, V2).

The closed enclosure 4 may have an inlet for supplying the electrolyzed water 5 not shown in the figure so that the electrolyzed water 5 can be added to a specified level when the level becomes low. Also, an outlet not shown in the figure for draining the electrolyzed water 5 may be provided for maintenance period. Also, a level sensor not shown may be provided for detecting the level of the electrolyzed water 5. In addition, electromagnetic valves not shown may be provided for adjusting supply or draining.

The hollow-fiber membrane 4 is, for example, a plurality of ion-exchange polymer in thread shape are bound to constitute a membrane film. The receptacle 2 has the inlet port 8 and the outlet port 9, and both of the ports are connected with both ends of the hollow-fiber membrane 4.

The closed enclosure 4 maintains the electrolyzed water 5 and an internal gas (for example air) enclosed above the surface level of the electrolyzed water 5. Also, a connecting port 4a is provided on upper sidewall of the closed enclosure 4 for connecting with the pump 10 (P2) via the valve (V2).

At the bottom of the external sleeve 6, an opening 7 is provided; the electrolyzed water 5 enters therefrom inside the external sleeve 6 whereby the electrolyzed water 5 is provided in contact with the hollow-fiber membrane 1.

The pumps 10 (P1, P2) are gas compressors having a pressure adjustable function for adjusting the pressure of inside the closed enclosure 4 and the pressure of the subject gas into the hollow-fiber membrane respectively. The pumps 10 (P1, P2) may be any type of compressors including gas compressing piston pump, diaphragm pump, or rotary pump etc.

Next, operation of the apparatus for adding humidity to a subject gas of the present invention is described. First, the subject gas, for example, air or nitrogen for adding humidity is supplied from the inlet port 8 formed on the receptacle 2. The subject gas runs through the hollow-fiber membrane 1.

The subject gas is sufficiently added humidity while it goes through the hollow-fiber membrane, which is in contact with the electrolyzed water 5. The humidity added gas goes out from the outlet port 9 formed on the receptacle 2.

At this period, when the pressures from the pumps 10 (P1, P2) are increased by activating the pumps, the electrolyzed water 5 is expedited to penetrate the hollow-fiber membrane 1 easier. As a result, amount of water element from the electrolyzed water 5 increases for adding to the subject gas while it is passing through the hollow-finer membrane.

On the other hand, when the pressure of internal gas in the closed enclosure is decreased by activating the pumps 10 (P1, P2), the electrolyzed water 5 is depressed so that it is retarded to penetrate the hollow-fiber membrane 1. As a result, amount of water element from the electrolyzed water 5 reduces for adding to the subject gas passing through the hollow-finer membrane.

During such period, the pressure of the pump 10 (P1) connected to the inlet 8 is determined equal to or larger than the pressure of the pump 10 (P2). By this setting, the subject gas in upper part of the hollow-fiber membrane 1 above the surface level 11 exposed to the internal pressure in the closed enclosure 4 is prevented from going out from the pressure of the pump 10 (P1) to the pressure of the pump 10 (P2).

According to the apparatus for adding humidity to the subject gas of the present invention, in order to obtain a specified humidity of the subject gas from the outlet 9 formed on the receptacle 2, pressure and a flow rate of the pump 10 (P1), and pressure and a flow rate of the pump 10 (2) can be adjusted respectively.

In addition, the surface level 11 of the electrolyzed water 5 can be adjustably supplied to a specified surface level for obtaining a specified degree of humidity. By this adjustment, if the surface level is adjusted to a high level, surface area of the hollow-fiber membrane 1 in contact with the electrolyzed water 5 becomes large to make a degree of humidity high, if the surface level is adjusted to a low level, surface area of the hollow-fiber membrane 1 in contact with the electrolyzed water 5 becomes small to make a degree of humidity low.

By selecting the combination of the above adjustable factors, according to the apparatus for adding humidity to a subject gas of the present invention, the apparatus provides a hollow-fiber membrane 1 for the subject gas passing therethrough, a closed enclosure 4 for maintaining a water element containing liquid 5 therein at a specified level 11, and the subject gas for passing through the hollow-fiber membrane 1, and pressures and flow rates adjustment means for adjusting pressures and flow rates of the subject gas to be supplied into the hollow membrane and the closed enclosure respectively.

By this structure, the internal pressure and a flow rate of the subject gas in the closed enclosure 4, and pressure and a flow rate of the subject gas passing through the hollow-fiber membrane can be adjusted, and in addition, by adjusting the surface level of 11 of the electrolyzed water 5, an amount of a water element out of the electrolyzed water 5 or a water element containing liquid for adding to the subject gas can be precisely adjusted. By this structure, an apparatus having the features of adjusting a degree of humidity easily and precisely for adding humidity at a specified degree to the subject gas can be obtained.

According to the apparatus for adding humidity to gas of the present invention, each of the pressures and flow rates adjustment means including the pumps 10 (P1, P2) having the pressure adjustable function connected to an inlet 8 of the hollow-fiber membrane and the inlet 4a of the closed enclosure 4 respectively, and valves (V1, V2) disposed between one of the pumps 10 (P1) and the hollow-fiber membrane 1, and between one of the pumps 10 (P2) and the closed enclosure 4. Internal pressures can be easily adjusted to specified pressures by adjusting the pumps 10 (P1, P2) respectively, and flow rates can be adjusted to specified rates by adjusting the valves (V1, V2) respectively.

According to the embodiment of the present invention, the electrolyzed water 5 is used, for example, as a water element containing liquid. The electrolyzed water 5 has a bactericidal property, thereby providing a clean addition of humidity.

According to the embodiment of the present invention, the hollow-fiber membrane 1 is bent at the center thereof to form a U-character shape, however the shape of the hollow-fiber membrane cane be in a form of straight shape. In this case, the external sleeve 6 and the closed enclosure 4 can be also in the form of straight shape without losing the advantage of the present invention.

While the present invention is described in terms of preferred embodiments, however the above embodiments describe examples of the invention, and therefore many modifications and variations are possible in light of the above teachings and within the scope of the appended claims.

## Claims

1. An apparatus for adding humidity to gas, comprising:
a hollow-fiber membrane (1) for subject gas passing therethrough,
a closed enclosure (4) for maintaining a water element containing liquid (5) therein at a specified level (11) wherein said hollow-fiber membrane (1) is installed in contact with said water element containing liquid (5),
**characterized by**:
pressures and flow rates adjustment means for adjusting pressures and flow rates of said subject gas to be supplied into said hollow membrane (1) and in said closed enclosure (4), respectively.

2. An apparatus for adding humidity to gas according to claim 1, wherein each of said pressures and flow rate adjustment means further comprises:
pumps (10; P1, P2) connected to an inlet (8) of said hollow-fiber membrane (1) and said closed enclosure (4), respectively, and valves (V1, V2) disposed between one of said pumps (10; P1, P2) and said hollow-fiber membrane (1), and between one of said pumps (10; P1, P2) and said closed enclosure (4).

3. An apparatus for adding humidity to gas according to any one of claim 1 or 2, further comprising:
an external sleeve (6) for covering said hollow-fiber membrane (1),
wherein openings (7, 12) are formed on said external sleeve (6).

4. An apparatus for adding humidity to gas according to claim 3, wherein said external sleeve (6) has a flexible property.

5. An apparatus for adding humidity to gas according to any one of claim 1 through 4, wherein said closed enclosure (4) is adapted for maintaining an electrolyzed water as said water element containing liquid (5).

6. A method of adding humidity to gas using an apparatus according to any one of claims 1 to 5, comprising the step of using an electrolyzed water as said water element containing liquid (5).

7. A method of adding humidity to gas using an apparatus according to any one of claims 1 to 5, comprising the step of adjusting a pressure of said subject gas for adding humidity to equal to or higher than an internal pressure in said closed enclosure (4)

## Patentansprüche

1. Vorrichtung zum Befeuchten von Gas, welche Folgendes aufweist:
eine Hohlfasermembran (1), um dort hindurch laufendes Gas zu behandeln,
eine geschlossene Umhüllung (4) zum Halten einer ein Wasserelement enthaltenden Flüssigkeit (5), auf einem festgelegten Niveau (11), wenn die Hohlfasermembran (1) in Kontakt mit der ein Wasserelement enthaltenden Flüssigkeit (5) ist,
**gekennzeichnet durch**
Druck- und Flussrateneinstellmittel zum Einstellen von Drücken und Flussraten des vorliegenden Gases, welches in die Hohlfasermembran (1) bzw. die geschlossene Umhüllung (4) geliefert werden soll.

2. Vorrichtung zum Befeuchten von Gas nach Anspruch 1, wobei jedes der Druck- und Flussrateneinstellmittel weiter Folgendes aufweist:
Pumpen (10; P1, P2), die mit einem Einlass (8) der Hohlfasermembran (1) bzw. der geschlossenen Umhüllung (4) verbunden sind, und Ventile (V1, V2), die zwischen einer der Pumpen (10; P1, P2) und der Hohlfasermembran (1) und zwischen einer der Pumpen (10; P1, P2) und der geschlossenen Umhüllung (4) angeordnet sind.

3. Vorrichtung zum Befeuchten von Gas nach einem der Ansprüche 1 oder 2, die weiter Folgendes aufweist:
eine äußere Hülse (6) zum Bedecken der Hohlfasermembran (1), wobei Öffnungen (7, 12) an der äußeren Hülse (6) geformt sind.

4. Vorrichtung zum Befeuchten von Gas nach Anspruch 3, wobei die äußere Hülse (6) eine flexible Eigenschaft hat.

5. Vorrichtung zum Befeuchten von Gas nach einem der Ansprüche 1 bis 4, wobei die geschlossene Umhüllung (4) geeignet ist, um ein elektrolysiertes Wasser als die ein Wasserelement enthaltende Flüssigkeit (5) zu enthalten.

6. Verfahren zum Befeuchten von Gas unter Verwendung einer Vorrichtung nach einem der Ansprüche 1 bis 5, welches den Schritt aufweist, elektrolysiertes Wasser als die ein Wasserelement enthaltende Flüssigkeit (5) zu verwenden.

7. Verfahren zum Befeuchten von Gas unter Verwendung einer Vorrichtung nach einem der Ansprüche 1 bis 5, welches den Schritt aufweist, einen Druck des vorliegenden Gases zum Befeuchten gleich oder höher einem Innendruck der geschlossenen Umhüllung (4) einzustellen.

## Revendications

1. Appareil pour ajouter de l'humidité à un gaz, comprenant :
une membrane à fibres creuses (1) pour y faire passer un gaz sujet,
une enceinte fermée (4) pour maintenir un liquide contenant un élément d'eau (5) à un niveau spécifié (11), dans laquelle la membrane à fibres creuses (1) est installée en contact avec le liquide contenant un élément d'eau (5), **caractérisé par** :
des moyens de réglage de pressions et de débits pour régler les pressions et les débits du gaz sujet à fournir dans la membrane creuse (1) et dans l'enceinte fermée (4), respectivement.

2. Appareil pour ajouter de l'humidité à un gaz selon la revendication 1, dans lequel chacun des moyens de réglage de pression et de débit comprend en outre :
des pompes (10 ; P1, P2) connectées à un accès d'entrée (8) de la membrane à fibres creuses (1) et de l'enceinte fermée (4), respectivement, et des vannes (V1, V2) disposées entre l'une des pompes (10 ; P1, P2) et la membrane à fibres creuses (1), et entre l'une des pompes (10; P1, P2) et l'enceinte fermée (4).

3. Appareil pour ajouter de l'humidité à un gaz selon l'une des revendications 1 ou 2, comprenant en outre :
un manchon extérieur (6) pour couvrir la membrane à fibres creuses (1),
dans lequel des ouvertures (7, 12) sont formées sur le manchon extérieur (6).

4. Appareil pour ajouter de l'humidité à un gaz selon la revendication 3, dans lequel le manchon extérieur (6) a une propriété flexible.

5. Appareil pour ajouter de l'humidité à un gaz selon l'une quelconque des revendications 1 à 4, dans lequel l'enceinte fermée (4) est adaptée pour maintenir de l'eau électrolysée en tant que ledit liquide contenant un élément d'eau (5).

6. Procédé pour ajouter de l'humidité à un gaz en utilisant un appareil selon l'une quelconque des revendications 1 à 5, comprenant une étape consistant à utiliser de l'eau électrolysée en tant que liquide contenant un'élément d'eau (5).

7. Procédé pour ajouter de l'humidité à un gaz en utilisant un appareil selon l'une quelconque des revendications 1 à 5, comprenant une étape consistant à régler la pression du gaz sujet pour ajouter de l'humidité, pour qu'elle soit égale ou supérieure à la pression interne dans l'enceinte fermée (4).
